# EUROPEAN PATENT APPLICATION

(11) **EP 3 388 511 A1**
(43) Date of publication of application: **17.10.2018**
(21) Application number: 17166388.3
(22) Date of filing: 12.04.2017
(51) Int. Cl.: C12N 5/077, C12N 5/0775

(54) **NEW USES OF MAMMALIAN MUSCLE DERIVED STEL CELLS**

(71) Applicant: Revatis SA, 4000 Liege (BE)
(72) Inventor: SERTEYN, Didier, 4000 Liège (BE); CEUSTERS, Justine, 4000 Liège (BE)
(74) Representative: Bosch, Henry

(57) **Abstract**

The present invention relates to a method for assessing the effects of a compound or composition on a mammal, comprising obtaining mammalian pluripotent muscle-derived mesenchymal stem cells or a population thereof or differentiated cells derived therefrom, exposing the said stem cells, stem cell population or differentiated cells derived therefrom to the relevant compound or composition, and assessing the effects on the said cells. The invention method is a reliable assessment method applicable to drug development, toxicity testing and optimized personalized medicine.

## Description

The present invention relates to new uses of mammalian stem cells derived from muscular tissue, more specifically in the area of pharmaceutical treatment as well as drug discovery and screening as well as toxicity testing.

Drug development is becoming increasingly costly, not the least because of enormous costs linked to the evaluation of the drug efficacy and the determination of adverse effects associated with relevant drug candidates. Conventional drug development processes suffer from a major drawback linked to the high level of attrition of candidate agents as a result of unforeseen adverse effects. There is hence a need for improved models to reduce costs and attrition in the drug development process.

Further, appropriate medical treatment of certain diseases, such as more specifically certain cancers and diseases linked to genetic insufficiency or degeneration, more and more tend to require treatment solutions tailored to the patient characterized by his unique genetic background; that is therapy with the right drug at the right dose in the right patient. More recently, stem cells have been used to evaluate the patient's genetic code and/or determine the patient's reaction to a particular treatment, in order to adapt the medical treatment to the patient's expected reactions to the relevant treatment.

Such methods inevitably require satisfactory availability of stem cells. The use of stem cells is still controversial and the collection of stem cells from a patient may be burdensome and/or painful. One may differentiate two major categories of stem cells in mammals: embryonic stem cells and adult stem cells. Embryonic stem cells are mostly not available for a given patient and in any case are associated with ethical problems. Adult stem cells may be found in various tissues of an adult body. Their role is to maintain and repair the tissue in which they are found. These cells are produced throughout the entire life time of an individual and may be found in a newborn as well as in a child or adult. Several types of adult stem cells are known: Hematopoietic stem cells (HSC) which form the basis for blood cells and play a major role in the immune system may be found in bone marrow and in the umbilical cord. Stem cells from the umbilical cord are mostly no longer available when an adult patient requires them. The usual sources of adult mesenchymal stem cells are extracted from bone marrow or adipose tissues and the collection is invasive and painful. , These mesenchymal stem cells (MSC) are pluripotent and can differentiate into bone, cartilage, fat, muscle etc. Induced pluripotent stem cells (IPSC) have been generated artificially from differentiated cells as fibroblasts. Introduction of four specific genes encoding transcription factors could convert adult cells into pluripotent stem cells.

WO2015/091210 discloses mammalian muscle-derived pluripotent mesenchymal stem cells that may be obtained at minimal invasive effort, such as by muscular micro-biopsy, and that are available in sufficient quantities. These mesenchymal stem cells (MSCs) are positive at least for CD105, preferably for CD44, CD90 and CD105, and negative for CD45, MHCII and CD29. These MSCs typically are furthermore positive for miR-128, miR-133B, and miR-802, slightly positive for miR-218 and negative for miR-656. It has also been found that these cells support a plurality of freeze-thawing cycles without loosing their pluripotency. It is further noted that for cell culture the muscular micro-biopsies are used as explants and progenitor cells appear spontaneously in due time. By doing so, the number of manipulations is reduced, avoiding potential sources of contamination. No external growth factors need to be added, since the growth factors that are naturally secreted by the muscle micro-biopsy (the explant) are sufficient. Muscle-derived cells are a mixture of subpopulations from different lineages and different developmental stages. On the basis of their density, related to their expression of specific molecular markers, and thanks to a (discontinuous) density gradient, three substantially pure subpopulations of pluripotent mesenchymal stem cells have been isolated out of the muscular explants. All three subpopulations comprise >90% of cells that are CD44 positive. For CD90, the rate of expression is 36% for 25-35% fraction, 48% for <15% fraction and 73% for 15-25% fraction. For CD105, the rate of expression is higher than 55%, preferably higher than 60% and may be as high as 80% or 85% or 90% or even 95% in the three populations. The three populations also show different CFU-F and proliferative properties (see WO2015/091210 Examples section).

It has now been found that the mesenchymal stem cells as disclosed in WO2015/091210 are particularly suited for screening and/or testing new pharmaceutical compounds as they are pluripotent without artificial induction and may be differentiated into many cell types, and are easily available. The collection of these cells, more specifically by a muscular micro-biopsy, is easy and does not involve any significant pain.

It has further been found that the stem cells disclosed in WO2015/091210 are particularly suitable for use in personalized medicine comprising treatment solutions tailored to the relevant patient, as they may be collected from the relevant patient at any time, may be differentiated into many cell types and may hence be appropriately evaluated for drug efficacy and adverse drug effects.

More specifically, the invention relates to a method for assessing the efficacy and/or adverse effects of a compound or composition, preferably a pharmaceutical compound or composition, on a mammal, comprising obtaining mammalian pluripotent muscle-derived mesenchymal stem cells or a population thereof or mesenchymal stem cells differentiated therefrom, exposing the said stem cells or stem cell population or cells differentiated therefrom to the relevant compound or composition and assessing the effects thereof on the said stem cells or cell population or differentiated cells, wherein the mammalian pluripotent muscle-derived stem cells are obtained by muscular micro-biopsy followed by density gradient centrifugation, and are positive at least for CD105 and negative for CD45, MHCII and CD29.

The mammalian pluripotent muscle-derived mesenchymal stem cells may be obtained by collecting a micro-biopsy from mammalian muscular tissue, possibly placing the micro-biopsy in a culture medium, collecting cells emerging from said culture medium, growing the cells obtained to near confluency, dissociating the cells obtained, and separating the mesenchymal stem cells from the remaining cells of the medium by density gradient fractionation.

Advantageously, the microbiopsy is obtained from skeletal muscle tissue, such as from muscles from the neck, shoulder, chest, back, tail, limbs, hindlimb, forelimb, hindquarters, hindleg etc. In the case of a mammal like a horse or human the microbiopsy is preferably obtained from triceps brachii muscle tissue, more preferably taken from the long head of the triceps brachii, or from the deltoid muscle.

In the case of a horse, for instance, the micro-biopsy may be collected at a depth of about 5 cm in the long head of the triceps brachii and may contain about 10 to about 30 mg, preferably more than about 12 mg or more than about 15 mg and/or less than about 25 mg or less than about 20 mg of tissue.

Depending on the mammal and skeletal muscle tissue source, the skilled person will be able to adapt the depth of microbiopsy and quantity of tissue sample to be extracted, on the basis of his general knowledge and/or by routine experimentation without undue burden.

The culture medium may comprise DMEM/F12 with about 20% fetal bovine serum, about 5ml penicillin (1000U/ml)-streptomycin (10000µg/ml), about 2.5ml amphotericin B (250µg/mL) and about 5ml HEPES or other specific medium without animal proteins (FBS free medium for example) Again, the culture medium may be adapted to the particular applications, if so required.

Preferably, the mammalian pluripotent muscle-derived stem cells are positive for CD44, CD90 and CD105 and negative for CD45, MHCII and CD29.

For the sake of completeness, it has been found that the relevant mammalian pluripotent muscle-derived stem cells may further be positive for miR-128, miR-133B, and miR-802. Further, they may be slightly positive for miR-218 and negative for miR-656.

Depending on the compound or composition to be evaluated, the isolated stem cells may be further differentiated into adipocytes, osteocytes, chondrocytes, myogenic cells, hematopoetic cells, endothelial cells, neural cells, cardiac cells, or hepatocytes by culturing the MSCs in an adequate adipogenic, osteogenic, chondrogenic, myogenic, hematopoetic, endothelial, neuronal, cardial, or hepatocytic differentiation medium respectively.

The effects of the relevant compound or composition to be tested, preferably a pharmaceutical compound or composition, may be determined in a manner known per se or within the general knowledge of the person skilled in the art. As an example, particularly in the case of pharmaceutical compounds or compositions, the effects of the relevant compound or composition may be assessed by comparison with a control conducted in the absence of the pharmaceutical compound or composition.

The method of the present invention may also find application in a personalized medical treatment method, wherein the said mammalian pluripotent muscle-derived stem cells or a population thereof or cells differentiated therefrom are derived from a patient, the method further comprising assessing the likelihood of a positive response by the patient by evaluating the level of the positive response of the said mammalian pluripotent muscle-derived stem cells or a population thereof.

The present invention offers an effective and promising alternative to the methods already described in the literature and provides the possibility of being carried out on living mammals due to the minimally invasive character of the stem cell collection. As is known, muscle-derived cells are a mixture of subpopulations from different lineages and different developmental stages. On the basis of their density, related to their expression of specific molecular markers, and thanks to a (discontinuous) density gradient, it is possible to select three substantially pure subpopulations of pluripotent mesenchymal stem cells out of the muscular explants. Cell culture may be initiated with a simple method: the muscular microbiopsies may be used as explants and progenitor cells appear spontaneously in due time. By doing so, the number of manipulations is reduced, avoiding potential sources of contamination, and no external growth factors need to be added, since the growth factors that are naturally secreted by the muscle microbiopsy (the explant) are sufficient.

Contrary to state of the art assessment methods making use of induced pluripotent stem cells (IPSC), the present invention further appears particularly suitable for evaluation of efficacy and/or adverse effects of a compound or composition on a patient by assessing the relevant effects on autologous pluripotent stem cells. The assessment therefore is more reliable (compared to IPSCs or non-autologous cells) as the result is less depending on genetic particularities or anomalies. This advantage is of particular relevance, more especially in the assessment of pharmacological effects against genetic disorders and in optimized personalized medicine.

One of the advantages here also is that said mesenchymal stem cells (MSCs) can form fibroblasts-like colonies in culture. In addition, unlike what is normally observed with other mesenchymal stem cell sources, said cells can differentiate into adipocytes, chondrocytes and osteocytes and support a plurality of freeze-thawing cycles without loss of their pluripotency.

In the present description, the term "pharmaceutical" (pharmaceutical compound, pharmaceutical composition) is understood to mean relating to drugs or pharmacy, and includes both, human and veterinary medicine.

In the present description, the term "about" as used herein when referring to a measurable value such as a parameter, an amount, a temporal duration, and the like, is meant to encompass variations of +/-10% or less, preferably +/-5% or less, more preferably +/-1% or less, and still more preferably +/-0.1% or less of and from the specified value, insofar such variations are appropriate to perform in the disclosed invention. It is to be understood that the value to which the modifier "about" refers is itself also specifically, and preferably, disclosed.

For general methods relating to the invention, reference is made to well-known textbooks, including, e.g., "Molecular Cloning: A Laboratory Manual, 2nd Ed." (Sambrook et al., 1989), Animal Cell Culture (R. I. Freshney, ed., 1987), the series Methods in Enzymology (Academic Press), Gene Transfer Vectors for Mammalian Cells (J. M. Miller Et M. P. Calos, eds., 1987); "Current Protocols in Molecular Biology and Short Protocols in Molecular Biology, 3rd Ed." (F. M. Ausubel et al., eds., 1987 Et 1995); Recombinant DNA Methodology II (R. Wu ed., Academic Press 1995), incorporated by reference herein

For further elaboration of general techniques useful in the practice of this invention, the practitioner can refer to standard textbooks and reviews in cell biology, tissue culture, and embryology. Included are "Teratocarcinomas and embryonic stem cells: A practical approach" (E. J. Robertson, ed., IRL Press Ltd. 1987); "Guide to Techniques in Mouse Development" (P. M. Wasserman et al. eds., Academic Press 1993); "Embryonic Stem Cell Differentiation in Vitro" (M. V. Wiles, Meth. Enzymol. 225:900, 1993); "Properties and uses of Embryonic Stem Cells: Prospects for Application to Human Biology and Gene Therapy" (P. D. Rathjen et al., al., 1993). Differentiation of stem cells is reviewed, e.g., in Robertson. 1997. Meth Cell Biol 75: 173; and Pedersen. 1998. Reprod Fertil Dev 10: 31, and U̅sas et al., 2011, incorporated by reference herein.

General techniques in cell culture and media collection are outlined in Large Scale Mammalian Cell Culture (Hu et al. 1997. Curr Opin Biotechnol 8: 148); Serum-free Media (K. Kitano. 1991. Biotechnology 17: 73); Large Scale Mammalian Cell Culture (Curr Opin Biotechnol 2: 375, 1991), Culture of animal cells: A manual of basic technique, Freshney, R.I. et al. 2005, 5th Edition, Wiley, New York, incorporated by reference herein.

The term "stem cell" refers generally to an unspecialised or relatively less specialised and proliferation-competent cell, which is capable of self-renewal, i.e., can proliferate without differentiation, and which or the progeny of which can give rise to at least one relatively more specialised cell type. The term encompasses stem cells capable of substantially unlimited self-renewal, i.e., wherein the progeny of a stem cell or at least part thereof substantially retains the unspecialised or relatively less specialised phenotype, the differentiation potential, and the proliferation capacity of the mother stem cell, as well as stem cells which display limited self-renewal, i.e., wherein the capacity of the progeny or part thereof for further proliferation and/or differentiation is demonstrably reduced compared to the mother cell. By means of example and not limitation, a stem cell may give rise to descendants that can differentiate along one or more lineages to produce increasingly relatively more specialised cells, wherein such descendants and/or increasingly relatively more specialised cells may themselves be stem cells as defined herein, or even to produce terminally differentiated cells, i.e., fully specialised cells, which may be post-mitotic.

The term "mesenchymal stem cell" or "MSC" as used herein refers to a mammalian adult, mesoderm-derived stem cell that is capable of generating cells of mesenchymal lineages, typically cells of two, preferably of three or more mesenchymal lineages, e.g., osteocytic (bone), chondrocytic (cartilage), myocytic (muscle), tendonocytic (tendon), fibroblastic (connective tissue), adipocytic (fat) and stromogenic (marrow stroma) lineage. Commonly, but without limitation, a cell may be considered MSC if it is capable of forming cells of each of the adipocytic, chondrocytic and osteocytic lineages, using standard, art-accepted differentiation conditions and cellular phenotype evaluation methods, e.g., as described in Pittenger et al. 1999 (Science 284: 143-7) or Barberi et al.,2005 (PLoS Med 2: e161), and U̅sas et al., 2011. The term MSC also encompasses the progeny of MSC, e.g., progeny obtained by *in vitro* or *ex vivo* propagation of MSC obtained from a biological sample of a subject.

The ISCT determined precisely the qualities cells must possess to be defined as mesenchymal stem cells (MSCs) as follows: the cells must be plastic-adherent, positive for the markers CD73, CD90 and CD105, negative for the markers CD14 (or CD11b), CD34, CD45, CD79a (or CD19) and MHC-II, and must exhibit the ability to differentiate into cells of mesodermal origin such as osteoblasts, chondroblasts and adipocytes (Dominici et al., 2006). The use of other MSC markers such as CD29 or CD44 was also reported (Pittenger et al., 1999). The mammalian MSC cells as used in accordance with the present invention hence are defined in that they express or co-express (i.e., are positive for) at least the mesenchymal marker CD105, and preferably also one or more of the following markers: CD44 and CD90. The mammalian MSC cells of the present invention are also defined in that they may express or co-express (i.e., are positive for) one or more of the following microRNAs: miR-128, miR-133B, miR-218 or miR-802. The mammalian MSC cells of the present invention are also defined in that they do not express miR-656.

The terms microRNA, miRNA, miR or eca-miR are used herein interchangeably, and refer to 19-25 nucleotides mature non-coding RNAs or precursors thereof, or fragments thereof, derived from endogenous genes of living organisms such as animals. Mature microRNAs are processed from longer hairpin-like precursors termed pre-microRNAs (pre-miRs) having a length of approximately 75 nucleotides.

Where a cell is said to be positive for a particular marker or microRNA, this means that a skilled person will conclude the presence or evidence of a distinct signal, e.g., antibody-detectable or detection by reverse transcription polymerase chain reaction, for that marker or microRNA when carrying out the appropriate measurement, compared to suitable controls. Where the method allows for quantitative assessment of the marker or microRNA, positive cells generate a signal that is significantly different from and higher or stronger than the control, e.g., but without limitation, at least 1.5-fold higher than such signal generated by control cells, e.g., at least 2-fold, at least 4-fold, at least 10-fold, at least 20-fold, at least 30-fold, at least 40-fold, at least 50-fold higher or even higher.

The expression of cell-specific markers can be detected using any suitable immunological technique known in the art, such as immuno-cytochemistry or affinity adsorption, Western blot analysis, FACS, ELISA, etc., or by any suitable biochemical assay of enzyme activity, or by any suitable technique of measuring the quantity of the marker mRNA, e.g., Northern blot, semi-quantitative or quantitative RT-PCR, etc.

The expression of microRNAs may be determined, for example, with an assay for global gene expression (e.g. using a microarray assay for microRNAs expression profiling analysis, a ready-to-use microRNA qPCR plate or RNA sequencing) or by specific detection assays, for example, but not limited to, quantitative PCR, quantitative reverse-transcription (real-time) PCR (qRT-PCR), locked nucleic acid (LNA) real-time PCR, or northern blotting. In particular, the measurement of the expression of a microRNA may be carried out with an oligonucleotide probe specific for the detection of said microRNA. Said oligonucleotide probe may bind directly and specifically to the microRNA, or may specifically reverse transcribe said microRNA. Alternatively, said oligonucleotide probe may bind a cDNA obtained from said microRNA. Said oligonucleotide probe may also amplify a cDNA obtained form said microRNA.

Nucleic and amino acid sequence data for marker proteins listed in this disclosure are generally known and can be obtained from public databases such as, among others, from the NIH "Protein Reviews on the Web" database (http://mpr.nci.nih.gov/prow/), the NIH "Entrez Gene" database (http://www.ncbi.nlm.nih.gov/sites/entrez?db=gene) or the Uniprot/Swissprot database (http://www.expasy.org/). Suitable detection reagents and methods for said markers can be designed either on the basis of such sequence information or, more commonly, are available commercially (e.g., labelled monoclonal antibody reagents).

The term "CD105" encompasses the antigen known as CD105, or its synonyms such as endoglin. CD105 is a membrane glycoprotein located on cell surfaces and is a known mesenchymal stem cell marker. As an example, the partial amino acid sequence of the equine CD105 antigen can be found in the Genbank database under accession number AGW16345.1.

The term "CD90" encompasses the antigen CD90, or its synonyms such as Thy-1 membrane glycoprotein. As an example, the amino acid sequence of the equine CD90 antigen can be found in the Genbank database under accession number ACG61223.1.

The term "CD44" encompasses the antigen generally known as CD44, or its synonyms such as Extracellular matrix receptor III, GP90 lymphocyte homing/adhesion receptor, HUTCH-I, Hermes antigen, Hyaluronate receptor, or Phagocytic glycoprotein 1. As an example, the amino acid sequence of the equine CD44 antigen can be found in the Genbank database under accession number CAA47331.1.

Exemplary commercially available antibody reagents for detection of said MSC markers include *inter alia* monoclonal antibodies anti-CD105-RPE (ABD Serotec), anti-CD44-APC (BD Pharmigen), and anti-CD90 (VMDR). Alternative antibodies that are specifically binding to CD105, CD44, or CD90 can be identified by the person skilled in the art.

MicroRNAs listed in this disclosure are generally known and can be obtained from public databases such as, among others, the miRBase database (http://www.mirbase.org). The term "miR-128" encompasses the microRNA known as miR-128 or its precursor. As an example, the nucleotide sequence of the equine miR-128 can be found in the miRBase database under accession number MI0012821. The term "miR-133B" encompasses the microRNA known as miR-133B or its precursor. As an example, the nucleotide sequence of the equine miR-133B can be found in the miRBase database under accession number MI0012844. The term "miR-656" encompasses the microRNA known as miR-656 or its precursor. As an example, the nucleotide sequence of the equine miR-656 can be found in the miRBase database under accession number MI0012915. The skilled person is well aware that microRNAs may be referred to by different names, or synonyms.

The term "cell population" generally refers to a grouping of cells. A cell population may consist of or may comprise at least a fraction of cells of a common type, or having characteristics in common. Such characteristics may include, without limitation, morphological characteristics, potential for differentiation (e.g., pluripotent, multipotent, unipotent, etc.; e.g., if multipotent or unipotent, ability to differentiate towards specific cell types), or the presence and/or level of one, two, three or more cell-associated markers, e.g., surface antigens. Such characteristics may thus define a cell population or a fraction thereof. Preferably, such a cell population is mesenchymal stem cell population, more preferably a substantially homogenous population of mesenchymal stem cells.

The term "substantially homogeneous" or "substantially pure" population of mesenchymal stem cells denotes a cell population comprising a fraction of MSCs as defined above, wherein said fraction in said cell population is at least 50%, e.g., at least 55%, preferably at least 60%, e.g., at least 65%, more preferably at least 70%, e.g., at least 75%, even more preferably at least 80%, e.g., at least 85%, most preferably at least 90%, e.g., at least 91%, at least 92%, at least 93%, at least 94%, at least 95%, at least 96%, at least 97%, at least 98%, at least 99%, or even close to or equal to 100%.

The expression "density gradient centrifugation" encompasses all types of cell-separation techniques or products encompassing the density-based separation of cells. Non-limiting examples can be density gradient centrifugation in a gradient of sucrose polymer, or colloidal silica. Non-limiting examples of commercially available gradients are: percoll (colloidal silica coated with polyvinylpyrrolidone or silane), ficoll (high molecular weight sucrose-polymers), Ficoll-Paque (Ficoll plus sodium diatrizoate and edetate calcium disodium), buoyant density solution (BDS, comprising colloidal silica), lymphoprep (sodium diatrizoate and polysaccharide), etc. It is clear that the skilled artisan will be able to select suitable gradients to separate the stem cells obtained with the method according to the present invention. Using the methods of the present invention, the mesenchymal stem cells (MSCs) are typically found in the <15%, 15-25%, or 25-35% Percoll density interfaces, after centrifugation at 1250 x g (25°C, 20min). Mesenchymal stem cells co-expressing the desired marker proteins can then be selected, enriched or isolated from the general population of isolated and optionally expanded cells by methods known *per se,* such as, for example, using fluorescence activated cell-sorting (FACS), magnetic-activated cell sorting (MACS), affinity-based technologies *inter alia* affinity chromatography, or the preplate technique and combinations thereof. Exemplary methods are reported in Wu et al., 2010 (cf. Cell Tissue Research, Jun;340(3):549-67).

Live cells having a desired expression profile are allowed to bind with reagents (most commonly immunological reagents such as, e.g., monoclonal antibodies) specific for the respective markers, wherein said reagents are in turn modified (e.g., by a fluorophore, or by immobilisation on magnetic particles or another type of stationary phase), such as to facilitate for selection or capture of cells bound by said reagents from cells not so bound. For general guidance on these methods, refer *inter alia* to Flow Cytometry and Cell Sorting, 2nd ed., by Andreas Radbruch (ed.), Springer 1999 (ISBN 3540656308); In Living Color: Protocols in Flow Cytometry and Cell Sorting, 1st ed., by RA Diamond and S Demaggio (eds.), Springer 2000 (ISBN 3540651497); Flow Cytometry Protocols (Methods in Molecular Biology), 2nd ed., by TS Hawley and RG Hawley (eds.), Humana Press 2004 (ISBN 1588292355); Affinity Separations: A Practical Approach, P Matejtschuk (ed.), Oxford University Press, 1997 (ISBN 0199635501); and Dainiak et al. 2007. Adv Biochem Eng Biotechnol 106: 1 - 18.

The expression "suitable culture medium" encompasses all cell-culturing media that support the survival and/or growth of the cells mesenchymal stem cells (MSCs) or mesenchymal stem cell populations. Non-limiting examples are: DF20, DMEM-Ham's F12, DMEM, Alpha-MEM etc., optionally supplemented with antifungal agents and buffers. As an example only, the following culture medium has been used: DF20 medium comprising: DMEM/F12 with about 20% fetal bovine serum, about 5ml penicillin (1000U/ml)-streptomycin (10000µg/ml), about 2.5ml amphotericin B (250µg/mL) and about 5ml HEPES. Other examples are CTS (commercial denomination) and Therapeak (commercial denomination) culture medium.

For differentiation into e.g. adipocytes, osteocytes and chondrocytes, the MSCs or mesenchymal stem cell populations of the invention where cultured in an adequate "differentiation medium". Said differentiation medium can for example be: for adipogenic differentiation: NH AdipoDiff Medium (Miltenyi Biotec); for chondrogenic differentiation: chondrocyte differentiation medium (NH ChondroDiff Medium; Miltenyi Biotec); for osteogenic differentiation: osteogenic medium (NH OsteoDiff Medium; Miltenyi Biotec). The media listed herein are merely shown as exemplary media, but the skilled person will be able to use any other commercial or specifically developed differentiation medium. Other examples of suitable differentiation media for other cells such as myogenic cells, hematopoetic cells, endothelial cells, neural cells, cardiac cells, or hepatocytes can be done by culturing the MSCs in an adequate myogenic, hematopoetic, endothelial, neuronal, cardial, or hepatocytic differentiation medium respectively, examples of which can e.g. be found in Usas et al., 2011.

Appropriate ways of "detaching", "dispersing", "dissociating" or "disassociating" cells are generally known in the art and may be used in the present invention. These involve, e.g., treatment with proteolytic enzymes, chelation of bivalent ions, mechanical disintegration, or combinations of any of the above. Preferably, said cell dissociation may involve enzymatic digestion, favourably using trypsin (e.g., as described above), optionally in combination with chelation of bivalent ions, favourably using EDTA (e.g., as described above), and/or mechanical dissociation of the so-treated cells. The latter may involve, e.g., repeated passing of the cells through a small bore pipette (e.g., a 1000µl micropipette tip) and/or pipetting out a stream of a suspension containing the cells against a solid surface (e.g., against the wall of the culture vessel). In this way a cell suspension comprising MSCs of the invention can be obtained.

The term "exposing" used herein includes all methods for subjecting the relevant cells to the potential effects of a compound or composition, including for instance a toxic agent or a pharmaceutical agent, as known in the relevant pharmaceutical field. Such methods include for example contacting relevant cells and relevant compound or composition in an appropriate medium.

The method of the present invention allows for evaluation of efficacy and/or adverse effects of a compound or composition on autologous cells of a mammal, thereby providing a reliable assessment method that is independent on genetic particularities or anomalies. As a consequence, the invention method is particularly suitable for the assessment of pharmacological effects against genetic disorders.

## Claims

1. A method for assessing the efficacy and/or adverse effects of a compound or composition, preferably a pharmaceutical compound or composition, on a mammal, comprising obtaining mammalian pluripotent muscle-derived mesenchymal stem cells or a population thereof or mesenchymal stem cells differentiated therefrom, exposing the said stem cells or stem cell population or mesenchymal stem cells differentiated therefrom to the relevant compound or composition and assessing the effects of the relevant compound or composition on the said stem cells or stem cell population, wherein the mammalian pluripotent muscle-derived stem cells are obtained by muscular micro-biopsy, followed by density centrifugation, and are positive for at least CD105 and negative for CD45, MHCII and CD29.

2. Method according to claim 1 wherein the mammalian pluripotent muscle-derived stem cells are positive for CD44, CD90 and CD105 and negative for CD45, MHCII and CD29.

3. Method according to claim 1 or 2 wherein the mammalian pluripotent muscle-derived stem cells further are positive for miR-128, miR-133B, and miR-802.

4. Method according to claim 3 wherein the mammalian pluripotent muscle-derived stem cells further are slightly positive for miR-218 and negative for miR-656.

5. Method according to any preceding claim wherein the effects of the pharmaceutical compound or composition are assessed by comparison with a control conducted in the absence of the relevant compound or composition.

6. Method according to any of claims 1 to 5 wherein the method is a drug screening method and the pharmaceutical compound or composition is tested on the said stem cells or stem cell population derived from a multitude of individuals, or on differentiated cells derived therefrom.

7. Method according to any of claims 1 to 5 wherein the said mammalian pluripotent muscle-derived stem cells or a population thereof are derived from a mammalian patient, the method further comprising assessing the likelihood of a positive response by the mammalian patient by evaluating the level of a positive response of the said mammalian pluripotent muscle-derived stem cells or a population thereof or differentiated cells derived therefrom.
